# EUROPEAN PATENT SPECIFICATION

(11) **EP 3 938 001 B1**
(45) Date of publication and mention of the grant of the patent: **29.07.2026**
(21) Application number: 20769968.7
(22) Date of filing: 11.03.2020
(51) Int. Cl.: A61L 27/56, A61F 2/02, A61M 5/00, A61K 35/39, A61K 38/28, A61K 9/00, A61K 47/34

(54) **CELL ENCAPSULATION DEVICES**
VORRICHTUNGEN ZUR VERKAPSELUNG VON ZELLEN
DISPOSITIFS D'ENCAPSULATION DE CELLULES

(30) Priority: 11.03.2019 US 201962816675 P
(43) Date of publication of application: 19.01.2022
(73) Proprietor: Encellin, Inc., San Francisco, California 94105 (US)
(72) Inventor: NYITRAY, Crystal, San Francisco, California 94105 (US); WEI, Grace, San Francisco, California 94105 (US)
(74) Representative: Avidity IP
(86) International application number: PCT/US2020/022063
(87) International publication number: WO 2020/185874

(56) References cited:
- EP-A1- 3 409 239
- WO-A1-2016/154333
- WO-A1-2016/154333
- WO-A2-2008/079997
- US-A1- 2014 081 070
- US-A1- 2014 081 070
- US-A1- 2018 185 550
- RYAN CHANG ET AL: "Nanoporous Immunoprotective Device for Stem-Cell-Derived [beta]-Cell Replacement Therapy", ACS NANO, AMERICAN CHEMICAL SOCIETY, US, vol. 11, no. 8, 22 August 2017 (2017-08-22), pages 7747 - 7757, XP009520328, ISSN: 1936-0851, [retrieved on 20170807], DOI: 10.1021/ACSNANO.7B01239
- NYITRAY, C. E. ET AL.: "Polycaprolactone Thin-Film Micro- and Nanoporous Cell - Encapsulation Devices", ACS NANO, vol. 9, no. 6, 2015, pages 5675 - 5682, XP055318977, DOI: 10.1021/acsnano.5b00679
- CHANG, R. ET AL.: "Nanoporous Immunoprotective Device for Stem- Cell -Derived beta- Cell Replacement Therapy", ACS NANO, vol. 11, 2017, pages 7747 - 7757, XP009520328, DOI: 10.1021/acsnano.7b01239

## Description

### BACKGROUND OF THE INVENTION

The transplantation of cells into a subject to produce therapeutic molecules may be an attractive alternative to the use of exogenous drugs. The encapsulation of cell transplants may, e.g., prevent the proliferation of the transplanted cells in the host, and may increase the viability of transplanted cells. The present disclosure provides methods and devices for improving the transplantation of cells into a subject, including improving the viability and functionality of the transplanted cells. Document WO 2016/154333 A1 discloses a device for cell encapsulation comprising a) a first polymer layer comprising a plurality of pores and one or more channels, wherein the one or more channels connect two adjacent pores of the plurality of pores, and wherein the one or more channels comprise an average connectivity diameter of less than about 500 nm; b) a second polymer layer comprising a plurality of pores and one or more channels, wherein the one or more channels connect two adjacent pores.

### SUMMARY OF THE INVENTION

According to the present invention there is provided a cell encapsulation device comprising: a) a first polymer layer comprising a plurality of pores having an average connectivity diameter of less than about 500 nm; and b) a second polymer layer comprising a plurality of pores having an average connectivity diameter of greater than about 100 µm; wherein the first polymer layer and the second polymer layer define a lumen for enclosing a plurality of cells. In some cases, the plurality of pores present in the first polymer layer have an average connectivity diameter from about 10 nm to about 200 nm. In some cases, the plurality of pores present in the first polymer layer have an average connectivity diameter from about 180 nm to about 220 nm. In some cases, the plurality of pores present in the second polymer layer have an average connectivity diameter from about 100 µm to about 500 µm. In some cases, the plurality of pores present in the second polymer layer have an average connectivity diameter from about 200 µm to about 400 µm. In some cases, the plurality of pores present in the first polymer layer have an average pore diameter from about 1 µm to about 5 µm. In some cases, the plurality of pores present in the second polymer layer have an average pore diameter from about 500 µm to about 3 mm. In some cases, the plurality of pores present in the second polymer layer have an average pore diameter from about 100 µm to about 1 mm. In some cases, the first polymer layer has a thickness from about 10 µm to about 200 µm. In some cases, the first polymer layer has a thickness from about 20 µm to about 50 µm. In some cases, the first polymer layer has a thickness from about 18 µm to about 22 µm. In some cases, the second polymer layer has a thickness from about 1 mm to about 5 mm. In some cases, the second polymer layer has a thickness from about 400 µm to about 600 µm. In some cases, the device further comprises third polymer layer having an average connectivity diameter of less than about 500 nm. In some cases, the device further comprises a port for introducing the plurality of cells into the lumen of the device. In some cases, the first polymer layer, the second polymer, the third polymer layer, or any combination thereof, comprises a biocompatible polymer selected from the group consisting of: polycaprolactone (PCL), polyvinylidene fluoride (PVDF), polytetrafluoroethylene (PTFE), polyethylene (PE), methacrylate polymer, polyethyleneimine, polyethyleneimine-dextran sulfate, poly(vinylsiloxane) ecopolymerepolyethyleneimine, phosphorylcholine, poly(ethyl methacrylate), polyurethane, poly(ethylene glycol) (PEG), poly(lactic-glycolic acid) (PLGA), hydroxyapatite, poly(lactic acid), polyhydroxyvalerte and copolymers thereof, polyhydroxybutyrate and copolymers thereof, polydiaxanone, polyanhydride, polycyanocrylate, poly(amino acids), poly(orthoesters), polyesters, collagen, gelatin, cellulose polymer, chitosans, alginates, laminin, and any combination thereof. In some cases, the biocompatible polymer is polycaprolactone. In some cases, the plurality of cells are selected from the group consisting of: thyroid cells, parathyroid cells, bone marrow cells, mesenchymal stem cells, stromal cells, pluripotent stem cells, induced pluripotent stem cells, embryonic stem cells, blood vessel cells, cells derived from adipose tissue, cells derived from bone marrow, intestinal cells or cells derived therefrom, islets or islet cells, Sertoli cells, beta cells, progenitors of islet cells, progenitors of beta cells, peripheral blood progenitor cells, stem cells or derivatives thereof isolated from adult tissue, retinal progenitor derivative cells, cardiac progenitor derivative cells, osteoprogenitor cells, neuronal progenitor cells, genetically transformed cells, and any combination thereof. In some cases, the first polymer layer and the second polymer layer are directly sealed along a periphery of the first polymer layer and the second polymer layer. In some cases, the device does not comprise a support or frame. In some cases, the first polymer layer, the second polymer layer, or both, are non-laminated polymer layers.

Also disclosed but not part of the present invention, is a method comprising: implanting a device according to any of the preceding into a subject having or suspected of having a disease or disorder, wherein the device comprises a plurality of therapeutic cells, thereby treating the disease or disorder. In some cases, the subject is a human. In some cases, the plurality of cells are islets or islet cells. In some cases, the subject has or is suspected of having diabetes. In some cases, the plurality of cells are allogeneic cells, xenogeneic cells, or autologous cells. In some cases, the device is implanted with a plurality of cells enclosed within the lumen. In some cases, the device is implanted without any cells enclosed within the lumen. In some cases, a plurality of cells are introduced into the lumen after the device is implanted into the subject for a period of time. In some cases, the period of time is at least 1 week. In some cases, therapeutic molecules are configured to be released from the device. In some cases, the therapeutic molecules diffuse out of the device. In some cases, the therapeutic molecules comprise insulin. In some cases, the implanting is selected from the group consisting of: subcutaneous, omentum, intraperitoneal, retroperitoneal, and intramuscular implanting. In some cases, the device does not induce a foreign body response in the subject. In some cases, the therapeutic cells embed within the second polymer layer. In some cases, the therapeutic cells attach to the second polymer layer.

### BRIEF DESCRIPTION OF THE DRAWINGS

The present invention relates to a cell encapsulation device as set forth in the appended claims. A better understanding of the features and advantages of the present invention will be obtained by reference to the following detailed description that sets forth illustrative embodiments, in which the principles of the invention are utilized, and the accompanying drawings of which:
**FIG. 1A** and **FIG. 1B** depict a non-limiting example of a cell encapsulation device according to embodiments of the disclosure.
**FIGS. 2A-2E** depict a polycaprolactone scaffold for cell seeding. **FIG. 2A** depict a polymer scaffold cut to a circular wafer. **FIG. 2B** depicts the flexibility of the polymer scaffold. **FIG. 2C** depicts the highly porous architecture within the matrix. **FIG. 2D** depicts a scanning electron micrograph of pores within the polymer scaffold. **FIG. 2E** depicts a scanning electron microscopy image of a cross section of the polymer scaffold showing thickness and pores within the matrix.
**FIGS. 3A-3C** depict another non-limiting example of a cell encapsulation device according to the embodiments of the disclosure. **FIG. 3A** depicts the macroporous layer of the device. **FIG. 3B** depicts the nanoporous layer of the device. **FIG. 3C** illustrates a cross-section view of the device.
**FIG. 4** illustrates a non-limiting example of a method of loading cells into the device according to **FIGS. 3A-3C****,** and the subsequent arrangement of the cells into the macroporous layer of the device.
**FIG. 5** depicts a non-limiting example of subcutaneous placement of a device of the disclosure into a C57BL/6 mouse.
**FIG. 6A** illustrates a non-limiting example of results of a non-fasting, intraperitoneal glucose tolerance test (i.p.GTT) on day 21 post-transplantation of the device. **FIG. 6B** depicts a non-limiting example of results from a glucose-stimulated insulin secretion assay.
**FIG. 7** depicts a cross-section view of a device of the disclosure integrated into the body of a mouse. Immunofluorescence detection of CD31 and alpha-smooth muscle action (α-SMA), as well as DAPI nuclear staining, demonstrate that the vasculature can grow around and into the device.
**FIG. 8A** and **FIG. 8B** depict images of islets loaded into a device of the disclosure and implanted subcutaneously into mice (at 4X magnification and 10X magnification, respectively).

### DETAILED DESCRIPTION OF THE INVENTION

Disclosed herein are devices for the encapsulation of biological cells. In some cases, the devices may be configured to be implanted into a subject. In some cases, a device of the disclosure may comprise a first polymer layer and a second polymer layer. In some cases, the first polymer layer and the second polymer layer may be in contact at a periphery of the device, thereby forming an enclosed space or lumen between the first polymer layer and the second polymer layer. In some cases, the lumen may be configured to enclose a population of biological cells. The devices described herein may be designed to contain therapeutic cells (e.g., within a lumen or internal compartment). The devices described herein may be designed such that, when transplanted into a biological subject, the cells contained within the device remain viable and/or functional for a period of time.

As used herein and in the appended claims, the singular forms "a," "an," and, "the" include plural referents unless the context clearly dictates otherwise. It is further noted that the claims can be drafted to exclude any optional element. As such, this statement is intended to serve as antecedent basis for use of such exclusive terminology as "solely," "only," and the like in connection with the recitation of claim elements, or use of a "negative" limitation.

Certain ranges or numbers are presented herein with numerical values being preceded by the term "about." The term "about" is used herein to mean plus or minus 1%, 2%, 3%, 4%, or 5% of the number that the term refers to.

As used herein, the terms "subject" and "individual," are used interchangeably and can be any animal, including mammals (e.g., a human or non-human animal).

As used herein, the terms "treat", "treating", or "treatment", include alleviating, abating, or ameliorating a disease or condition symptoms, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition either prophylactically and/or therapeutically.

As used herein, the term "connectivity diameter" refers to the diameter of the open space or the channel connecting two adjacent pores. As used herein, the term "pore diameter" refers to the distance between two opposite walls of a pore.

As used herein, the term "encapsulated," refers to cells that are contained or enclosed within a device of the disclosure. For example, encapsulated cells may refer to cells that are contained within a lumen or an internal space of a device of the disclosure. In another example, encapsulated cells may refer to cells that are contained within a membrane of the device (e.g., embedded within or attached to a macroporous scaffold of the device).

As used herein, the term "lumen" refers to an internal space or volume of a device of a disclosure. In some cases, a lumen is created by sealing the periphery of a first polymer layer and a second polymer layer, thereby generating an internal space or volume for encapsulating cells. The term "lumen" as used herein also encompasses the space where cells embed or attach within the device, for example, the space (e.g., pores, channels, etc.) within a macroporous scaffold of the device.

Unless defined otherwise, all technical and scientific terms used herein have the same meaning as commonly understood by one of ordinary skill in the art to which the disclosure, e.g., methods and compositions described herein, belong. Although any methods and materials similar or equivalent to those described herein can also be used in the practice or testing of the methods and compositions described herein, representative illustrative methods and materials are now described.

In some aspects of the disclosure, the device may comprise a first polymer layer. In some cases, the first polymer layer may be a polymer membrane or a polymer film. The first polymer layer may comprise a plurality of pores. In some cases, the first polymer layer is a nanoporous polymer layer.

In some cases, the plurality of pores present in the first polymer layer may have an average connectivity diameter. In some cases, the plurality of pores present in the first polymer layer may have an average connectivity diameter ranging from about 10 nm to about 2 µm. For example, the plurality of pores present in the first polymer layer may have an average connectivity diameter ranging from about 20 nm to about 300 nm, from about 30 nm to about 300 nm, from about 10 nm to about 200 nm, from about 20 nm to about 200 nm, from about 30 nm to about 200 nm, from about 10 nm to about 100 nm, from about 20 nm to about 100 nm, from about 30 nm to about 100 nm, from about 30 nm to about 50 nm, from about 30 nm to about 40 nm, from about 20 nm to about 250 nm, from about 20 nm to about 150 nm, from about 25 to about 150 nm, from about 200 nm to about 500 nm, from about 350 nm to about 500 nm, from about 500 nm to about 1.5 µm, or from about 1.0 µm to about 2.0 µm. In some cases, the plurality of pores in the first polymer layer may have an average connectivity diameter of about 10 nm, about 20 nm, about 30 nm, about 40 nm, about 50 nm, about 60 nm, about 70 nm, about 80 nm, about 100 nm, about 110 nm, about 120 nm, about 130 nm, about 140 nm, about 150 nm, about 160 nm, about 170 nm, about 180 nm, about 190 nm, about 200 nm, about 210 nm, about 220 nm, about 230 nm, about 240 nm, about 250 nm, about 260 nm, about 270 nm, about 280 nm, about 290 nm, about 300 nm, about 310 nm, about 320 nm, about 330 nm, about 340 nm, about 350 nm, about 360 nm, about 370 nm, about 380 nm, about 390 nm, about 400 nm, about 410 nm, about 420 nm, about 430 nm, about 440 nm, about 450 nm, about 460 nm, about 470 nm, about 480 nm, about 490 nm, about 500 nm, about 550 nm, about 600 nm, about 650 nm, about 700 nm, about 750 nm, about 800 nm, about 850 nm, about 900 nm, about 950 nm, about 1.0 µm, about 1.1 µm, about 1.2 µm, about 1.3 µm, about 1.4 µm, about 1.5 µm, about 1.6 µm, about 1.7 µm, about 1.8 µm, about 1.9 µm, or about 2.0 µm. In one aspect, the average connectivity diameter may be about 200 nm. In another aspect, the average connectivity diameter may be about 2.0 µm.

In various aspects, the first polymer layer may have an average connectivity diameter of less than about 2.0 µm, less than about 1.9 µm, less than about 1.8 µm, less than about 1.7 µm, less than about 1.6 µm, less than about 1.5 µm, less than about 1.4 µm, less than about 1.3 µm, less than about 1.2 µm, less than about 1.1 µm, less than about 1.0 µm, less than about 975 nm, less than about 950 nm, less than about 925 nm, less than about 900 nm, less than about 875 nm, less than about 850 nm, less than about 825 nm, less than about 800 nm, less than about 775 nm, less than about 750 nm, less than about 725 nm, less than about 700 nm, less than about 675 nm, less than about 650 nm, less than about 625 nm, less than about 600 nm, less than about 575 nm, less than about 550 nm, less than about 525 nm, less than about 500 nm, less than about 475 nm, less than about 450 nm, less than about 425 nm, less than about 400 nm, less than about 375 nm, less than about 350 nm, less than about 325 nm, less than about 300 nm, less than about 275 nm, less than about 250 nm, less than about 225 nm, less than about 200 nm, less than about 175 nm, less than about 150 nm, less than about 125 nm, less than about 100 nm, less than about 75 nm, less than about 50 nm, less than about 25 nm, less than about 20 nm, or less than about 10 nm.

In some aspects of the disclosure, the plurality of pores present in the first polymer layer may have an average pore diameter. In some cases, the average pore diameter may range from about 1 µm to about 5 µm. For example, the plurality of pores present in the first polymer layer may have an average pore diameter ranging from about 1 µm to about 4.5 µm, from about 1 µm to about 4 µm, from about 1 µm to about 3.5 µm, from about 1 µm to about 3 µm, from about 1 µm to about 2.5 µm, from about 1 µm to about 2 µm, from about 1.5 µm to about 5 µm, from about 2 µm to about 5 µm, from about 2.5 µm to about 5 µm, from about 3 µm to about 5 µm, from about 3.5 µm to about 5 µm, or from about 4 µm to about 5 µm. In some cases, the plurality of pores present in the first polymer layer may have an average pore diameter of about 1 µm, about 1.5 µm, about 2 µm, about 2.5 µm, about 3 µm, about 3.5 µm, about 4 µm, about 4.5 µm, or about 5 µm. In some cases, the first polymer layer may have an average pore diameter of about 2 µm.

In various aspects of the disclosure, the average pore diameter may be less than about 5 µm, less than about 4.5 µm, less than about 4 µm, less than about 3.5 µm, less than about 3 µm, less than about 2.5 µm, less than about 2 µm, less than about 1.5 µm, or less than about 1 µm.

In some cases, the plurality of pores present in the first layer may have an average connectivity diameter that is smaller than the average pore size. In other cases, the plurality of pores present in the first layer may have an average connectivity diameter that is the same or substantially the same as the average pore size.

The first polymer layer may have a thickness. In some cases, the first polymer layer may have a thickness of about 200 µm or less, e.g., about 200 µm, about 190 µm, about 180 µm, about 170 µm, about 160 µm, about 150 µm, about 140 µm, about 130 µm, about 120 µm, about 110 µm, about 100 µm, about 90 µm, about 80 µm, about 70 µm, about 60 µm, about 50 µm, about 40 µm, about 30 µm, about 29 µm, about 28 µm, about 27 µm, about 26 µm, about 25 µm, about 24 µm, about 23 µm, about 22 µm, about 21 µm, about 20 µm, about 19 µm, about 18 µm, about 17 µm, about 16 µm, about 15 µm, about 14 µm, about 13 µm, about 12 µm, about 11 µm, or about 10 µm. In some cases, the thickness of the first polymer layer may be less than about 10 µm. In some cases, the thickness of the first polymer layer may be greater than about 10 µm but less than about 30 µm. In some cases, the thickness of the first layer may be greater than about 18 µm but less than about 22 µm. In some cases, the thickness of the first polymer layer may be greater than about 30 µm. In some cases, the thickness of the first polymer layer may be from about 10 µm to about 200 µm, from about 20 µm to about 50 µm, from about 50 µm to about 300 µm, or from about 100 µm to about 500 µm. In some cases, the thickness of the first polymer layer may be from about 10 µm to about 50 µm. In some cases, the thickness of the first polymer layer is about 20 µm.

The first polymer layer may comprise a biocompatible polymer. Biocompatible polymers may include, without limitation, polycaprolactone (PCL), polytetrafluoroethylene (PTFE), polyethylene (PE), polyvinylidene fluoride (PVDF), methacrylate polymers, polyethylene-imine and dextran sulfate, poly(vinylsiloxane) ecopolymerepolyethyleneimine, phosphorylcholine, poly(ethyl methacrylate), polyurethane, poly(ethylene glycol), poly(lactic-glycolic acid), hydroxyapatite, poly(lactic acid), polyhydroxyvalerte and copolymers, polyhydroxybutyrate and copolymers, polydiaxanone, polyanhydrides, polycyanocrylates, poly(amino acids), poly(orthoesters), polyesters, silicone, collagen, gelatin, cellulose polymers, chitosans, laminin, and alginates, or combinations thereof. In some cases, the first polymer layer may comprise polycaprolactone. In some cases, the biocompatible polymer may be biodegradable (e.g., dissolvable in a biological environment). In some cases, the first polymer layer may be manufactured by weaving, by casting, by extrusion, by deposition, by an emulsion process, and the like. In other cases, the first polymer layer may be manufactured by electrospinning processes or templating processes.

In some aspects of the disclosure, the device may comprise a second polymer layer. In some cases, the second polymer layer may be a polymer membrane or a polymer film. The second polymer layer may comprise a plurality of pores. In some cases, the second polymer layer may be a macroporous polymer layer. In some cases, the second polymer layer is a sponge or sponge-like. In some cases, the second polymer layer is a mesh.

The plurality of pores present in the second polymer layer may have an average connectivity diameter. In some cases, the plurality of pores present in the second polymer layer may have an average connectivity diameter ranging from about 50 µm to about 3 mm. For example, the plurality of pores present in the second layer may have an average connectivity diameter ranging from about 50 µm to about 1 mm, from about 200 µm to about 1.5 mm, from about 300 µm to about 2 mm, from about 400 µm to about 2.5 mm, from about 500 µm to about 3 mm, from about 100 µm to about 500 µm, from about 500 µm to about 1.5 mm, or from about 1.5 mm to about 3 mm. In some cases, the plurality of pores present in the second polymer layer may have an average connectivity diameter of about 50 µm, about 100 µm, about 150 µm, about 200 µm, about 250 µm, about 300 µm, about 350 µm, about 400 µm, about 450 µm, about 500 µm, about 550 µm, about 600 µm, about 650 µm, about 700 µm, about 750 µm, about 800 µm, about 850 µm, about 900 µm, about 950 µm, about 1.0 mm, about 1.1 mm, about 1.2 mm, about 1.3 mm, about 1.4 mm, about 1.5 mm, about 1.6 mm, about 1.7 mm, about 1.8 mm, about 1.9 mm, about 2.0 mm, about 2.1 mm, about 2.2 mm, about 2.3 mm, about 2.4 mm, about 2.5 mm, about 2.6 mm, about 2.7 mm, about 2.8 mm, about 2.9 mm, or about 3.0 mm.

In some cases, the second polymer layer may have an average connectivity diameter of greater than about 50 µm, greater than about 100 µm, greater than about 150 µm, greater than about 200 µm, greater than about 250 µm, greater than about 300 µm, greater than about 350 µm, greater than about 400 µm, greater than about 450 µm, greater than about 500 µm, greater than about 550 µm, greater than about 600 µm, greater than about 650 µm, greater than about 700 µm, greater than about 750 µm, greater than about 800 µm, greater than about 850 µm, greater than about 900 µm, greater than about 950 µm, greater than about 1.0 mm, greater than about 1.1 mm, greater than about 1.2 mm, greater than about 1.3 mm, greater than about 1.4 mm, greater than about 1.5 mm, greater than about 1.6 mm, greater than about 1.7 mm, greater than about 1.8 mm, greater than about 1.9 mm, greater than about 2.0 mm, greater than about 2.1 mm, greater than about 2.2 mm, greater than about 2.3 mm, greater than about 2.4 mm, greater than about 2.5 mm, greater than about 2.6 mm, greater than about 2.7 mm, greater than about 2.8 mm, greater than about 2.9 mm, or greater than about 3.0 mm.

The plurality of pores present in the second polymer layer may have an average pore diameter. In some cases, the plurality of pores present in the second polymer layer may have an average pore diameter ranging from about 100 µm to about 3 mm. For example, the plurality of pores present in the second layer may have an average pore diameter ranging from about 100 µm to about 1 mm, from about 200 µm to about 1.5 mm, from about 300 µm to about 2 mm, from about 400 µm to about 2.5 mm, from about 500 µm to about 3 mm, from about 100 µm to about 500 µm, from about 500 µm to about 1.5 mm, or from about 1.5 mm to about 3 mm. In some cases, the plurality of pores present in the second polymer layer may have an average pore diameter of about 100 µm, about 150 µm, about 200 µm, about 250 µm, about 300 µm, about 350 µm, about 400 µm, about 450 µm, about 500 µm, about 550 µm, about 600 µm, about 650 µm, about 700 µm, about 750 µm, about 800 µm, about 850 µm, about 900 µm, about 950 µm, about 1.0 mm, about 1.1 mm, about 1.2 mm, about 1.3 mm, about 1.4 mm, about 1.5 mm, about 1.6 mm, about 1.7 mm, about 1.8 mm, about 1.9 mm, about 2.0 mm, about 2.1 mm, about 2.2 mm, about 2.3 mm, about 2.4 mm, about 2.5 mm, about 2.6 mm, about 2.7 mm, about 2.8 mm, about 2.9 mm, or about 3.0 mm. The plurality of pores present in the second polymer layer may be organized stochastically.

In some cases, the second polymer layer may have an average pore diameter of greater than about 100 µm, greater than about 150 µm, greater than about 200 µm, greater than about 250 µm, greater than about 300 µm, greater than about 350 µm, greater than about 400 µm, greater than about 450 µm, greater than about 500 µm, greater than about 550 µm, greater than about 600 µm, greater than about 650 µm, greater than about 700 µm, greater than about 750 µm, greater than about 800 µm, greater than about 850 µm, greater than about 900 µm, greater than about 950 µm, greater than about 1.0 mm, greater than about 1.1 mm, greater than about 1.2 mm, greater than about 1.3 mm, greater than about 1.4 mm, greater than about 1.5 mm, greater than about 1.6 mm, greater than about 1.7 mm, greater than about 1.8 mm, greater than about 1.9 mm, greater than about 2.0 mm, greater than about 2.1 mm, greater than about 2.2 mm, greater than about 2.3 mm, greater than about 2.4 mm, greater than about 2.5 mm, greater than about 2.6 mm, greater than about 2.7 mm, greater than about 2.8 mm, greater than about 2.9 mm, or greater than about 3.0 mm.

The second polymer layer may have a thickness. In some cases, the second polymer layer has a thickness from about 200 µm to about 1 mm. For example, the second polymer layer may have a thickness from about 200 µm to about 500 µm, from about 250 µm to about 450 µm, from about 300 µm to about 500 µm, from about 350 µm to about 600 µm, from about 400 µm to about 800 µm, from about 550 µm to about 950 µm, from about 500 µm to about 1 mm. In some cases, the second polymer layer may have a thickness of about 200 µm, about 250 µm, about 300 µm, about 350 µm, about 400 µm, about 450 µm, about 500 µm, about 550 µm, about 600 µm, about 650 µm, about 700 µm, about 750 µm, about 800 µm, about 850 µm, about 900 µm, about 950 µm, or about 1 mm. In some cases, the second polymer layer may have a thickness of about 500 µm.

In some cases, the second polymer layer may have a thickness from about 1 mm to about 5 mm. For example, the second polymer layer may have a thickness from about 1 mm to about 5 mm, from about 2 mm to about 4 mm, about 3 mm to about 5 mm, from about 1 mm to about 3 mm, from about 2.5 mm to about 5 mm, or from about 1.5 mm to about 4.5 mm. In some cases the second polymer layer may have a thickness of about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2.0 mm, about 2.1 mm, about 2.2 mm, about 2.3 mm, about 2.4 mm, about 2.5 mm, about 2.6 mm, about 2.7 mm, about 2.8 mm, about 2.9 mm, about 3.0 mm, about 3.1 mm, about 3.2 mm, about 3.3 mm, about 3.4 mm, about 3.5 mm, about 3.6 mm, about 3.7 mm, about 3.8 mm, about 3.9 mm, about 4.0 mm, about 4.1 mm, about 4.2 mm, about 4.3 mm, about 4.4. mm, about 4.5 mm, about 4.6 mm, about 4.7 mm, about 4.8 mm, about 4.9 mm, or about 5.0 mm. In some cases, the second polymer layer may have a thickness of about 2 mm.

The second polymer layer may have a pore density. In some cases, the second polymer layer may have a pore density from about 0.01 g/cm³ to about 0.1 g/cm³. For example, the second polymer layer may have a pore density of about 0.01 g/cm³, about 0.02 g/cm³, about 0.03 g/cm³, about 0.04 g/cm³, about 0.05 g/cm³, about 0.06 g/cm³, about 0.07 g/cm³, about 0.08 g/cm³, about 0.09 g/cm³, or about 0.1 g/cm³.

The second polymer layer may comprise a biocompatible polymer. Biocompatible polymers may include, without limitation, polycaprolactone (PCL), polytetrafluoroethylene (PTFE), polyethylene (PE), polyvinylidene fluoride (PVDF), methacrylate polymers, polyethylene-imine and dextran sulfate, poly(vinylsiloxane) ecopolymerepolyethyleneimine, phosphorylcholine, poly(ethyl methacrylate), polyurethane, poly(ethylene glycol), poly(lactic-glycolic acid), hydroxyapatite, poly(lactic acid), polyhydroxyvalerte and copolymers, polyhydroxybutyrate and copolymers, polydiaxanone, polyanhydrides, polycyanocrylates, poly(amino acids), poly(orthoesters), polyesters, collagen, gelatin, cellulose polymers, chitosans, laminin, and alginates, or combinations thereof. In some cases, the second polymer layer may comprise polycaprolactone (PCL). In some cases, the biocompatible polymer may be biodegradable (e.g., dissolvable in a biological environment). In some cases, the second polymer layer may be manufactured by weaving, by extrusion, by casting, by deposition, by an emulsion process, and the like. In other cases the second polymer layer may be manufactured by electrospinning processes or templating processes.

In some aspects, the second polymer layer may act as a scaffold for biological cells within the lumen of the device. For example, biological cells contained within the lumen of the device may attach to the surface of and/or embed within the pores or channels of the second polymer layer. In some cases, the surface of the second polymer layer may be coated with one or more agents (e.g., a growth factor) that promote survival and/or growth and/or protection of the biological cells within the device. In some cases, the second polymer layer may promote vascularization of the cells encapsulated within the device. For example, the second polymer layer may allow the blood vessels to grow into or penetrate the second polymer layer, such that the blood vessels are in close proximity to the biological cells encapsulated within the device. The blood vessels penetrating the second polymer layer may provide a supply of oxygen and/or nutrients to the cells encapsulated within the device and may promote viability of the cells within the device.

In some cases, the devices of the disclosure may increase the viability of encapsulated cells relative to transplanted naked cells (e.g., cells not encapsulated within a device). The devices described herein may be particularly well suited for encapsulating cells that have a preference for attaching to or embedding within a substrate, for example, cells that have a preferred three-dimensional architecture or arrangement. In particular examples, the macroporous scaffold of the device provides a substrate for cells to embed or attach thereon. In some cases, the encapsulated cells embedded within or attached to the macroporous scaffold may have increased viability as compared to encapsulated cells that are not embedded or attached to a scaffold (e.g., free-floating in a lumen of a device).

In various aspects, the first polymer layer and the second polymer layer may each be non-laminated polymer layers. In other words, each of the first polymer layer and the second polymer layer may consist of a single polymer layer.

In some aspects, the device of the disclosure may further comprise a third polymer layer. In some cases, the third polymer layer may comprise a plurality of pores. In some cases, the plurality of pores may have average connectivity diameters and/or average pore diameters of those disclosed for the first polymer layer. In some cases, the third polymer layer is a nanoporous polymer layer. In some cases, the first and third polymer layers have the same or similar average connectivity diameters and/or average pore diameters. In some cases, the first and third polymer layers have different average connectivity diameters and/or average pore diameters. In some cases, the third polymer layer may be composed of a biocompatible polymer, such as any biocompatible polymer disclosed for the first polymer layer. In some cases, the first and third polymer layers are composed of the same biocompatible polymer material. In some cases, the first and third polymer layers are composed of different biocompatible polymer materials.

In such devices comprising a third polymer layer, the third polymer layer may be in contact at a periphery of the device with the first polymer layer, or the third polymer layer may be in contact at the periphery of the device with the first polymer layer and the second polymer layer, thereby forming an enclosed space or lumen between the first polymer layer and the third polymer layer. In such cases, the second polymer layer may be enclosed within the device (e.g., within the lumen, or lining the third polymer layer).

In various aspects, a device of the disclosure may have a total thickness of about 200 µm to about 5 mm. For example, a device of the disclosure may have a total thickness of about 200 µm, about 250 µm, about 300 µm, about 350 µm, about 400 µm, about 450 µm, about 500 µm, about 550 µm, about 600 µm, about 650 µm, about 700 µm, about 750 µm, about 800 µm, about 850 µm, about 900 µm, about 950 µm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2.0 mm, about 2.1 mm, about 2.2 mm, about 2.3 mm, about 2.4 mm, about 2.5 mm, about 2.6 mm, about 2.7 mm, about 2.8 mm, about 2.9 mm, about 3.0 mm, about 3.1 mm, about 3.2 mm, about 3.3 mm, about 3.4 mm, about 3.5 mm, about 3.6 mm, about 3.7 mm, about 3.8 mm, about 3.9 mm, about 4.0 mm, about 4.1 mm, about 4.2 mm, about 4.3 mm, about 4.4 mm, about 4.5 mm, about 4.6 mm, about 4.7 mm, about 4.8 mm, about 4.9 mm, or about 5.0 mm.

In some cases, a device of the disclosure may have a surface area of about 1 cm² to about 100 cm². For example, a device of the disclosure may have a surface area of about 1 cm², about 5 cm², about 10 cm², about 15 cm², about 20 cm², about 25 cm², about 30 cm², about 35 cm², about 40 cm², about 45 cm², about 50 cm², about 55 cm², about 60 cm², about 65 cm², about 70 cm², about 75 cm², about 80 cm², about 85 cm², about 90 cm², about 95 cm², or about 100 cm².

In some cases, the devices provided herein may have a two-dimensional planar shape. For example, the two-dimensional planar shape may be, without limitation, circular, elliptical, square, or rectangular. In some cases, the devices provided herein may have a three-dimensional shape. For example, the three-dimensional shape may be, without limitation, spherical, conical, cuboidal, cylindrical, triangular, hexagonal, tetrahedrical, pyramidal, or octagonal.

In some cases, the devices provided herein may be used to transplant a variety of different cell types into a biological subject. In some cases, the biological cells may be eukaryotic cells. In some cases, the biological cells may be prokaryotic cells. Generally, the biological cells may be derived from any source. In some cases, the cells may be bacterial cells, yeast cells, insect cells, or fungal cells. In some cases, the cells may be mammalian cells. Non-limiting examples of mammalian cell types that may be used include: thyroid cells, parathyroid cells, bone marrow cells, mesenchymal stem cells, stromal cells, pluripotent stem cells, induced pluripotent stem cells, embryonic stem cells, blood vessel cells, cells derived from adipose tissue, cells derived from bone marrow, intestinal cells or cells derived therefrom, islets or islet cells, Sertoli cells, beta cells, progenitors of islet cells, progenitors of beta cells, peripheral blood progenitor cells, stem cells or derivatives thereof isolated from adult tissue, retinal progenitor derivative cells, cardiac progenitor derivative cells, osteoprogenitor cells, neuronal progenitor cells, genetically transformed cells, or any combination thereof. In some cases, the cells may be islets or islet cells. In some cases, the cells may be beta-cells or beta-like cells. In some cases, the cells may be stem cells. Derivative cells may include isolated subsets of primary tissue cells in an unaltered state or modified through culture conditions or introduction of materials intracellularly. In some cases, the cells may be allogeneic cells. In some cases, the cells may be autologous cells. In some cases, the cells may be xenogeneic cells. In some cases, the cells may be cells that have been engineered to express one or more recombinant proteins.

In some cases, the cells may be derived from a human. In other cases, the cells may be derived from a non-human animal, including, but not limited to, a non-human primate, a livestock animal, a domestic pet (e.g., a dog, a cat, a sheep, a cow, a horse), or a laboratory animal. For example, a non-human animal can be an ape (e.g., a chimpanzee, a baboon, a gorilla, or an orangutan), an old world monkey (e.g., a rhesus monkey), a new world monkey, a dog, a cat, a bison, a camel, a cow, a deer, a pig, a donkey, a horse, a mule, a lama, a sheep, a goat, a buffalo, a reindeer, a yak, a mouse, a rat, a rabbit, a whale, or any other non-human animal. The encapsulated cells may be from the subject (e.g., autologous cells), from another donor (e.g., allogeneic cells) or from other species (e.g., xenogeneic cells). In a non-limiting example, the cells may be xenogeneic islets or islet cells (e.g., from a pig) that are transplanted into a human subject.

The cells can be introduced into the lumen of the device and the device may be immediately (within a day) implanted into a subject. Alternatively, the cells may be introduced into the lumen of the device and the device containing the cells encapsulated therein can be cultured for a period of time, e.g., greater than one day, to allow for the cells to proliferate and/or embed into the scaffold of the device, prior to implantation. In some cases, the cells can be cultured for up to 12 hours, up to 1 day, up to 2 days, up to 3 days, up to 4 days, up to 5 days, up to 6 days, up to 7 days, or greater than 7 days, before the device is implanted into the subject.

In some cases, the number of cells introduced into the lumen of the device is at least 100 cells, at least 200 cells, at least 300 cells, at least 400 cells, at least 500 cells, at least 1000 cells, at least 2000 cells, at least 3000 cells, at least 4000 cells, at least 5000 cells, at least 10,000 cells, at least 20,000 cells, at least 30,000 cells, at least 40,000 cells, at least 50,000 cells, at least 60,000 cells, at least 70,000 cells, at least 80,000 cells, at least 90,000 cells, at least 100,000 cells, at least 200,000 cells, at least 300,000 cells, at least 400,000 cells, at least 500,000 cells, at least 600,000 cells, at least 700,000 cells, at least 800,000 cells, at least 900,000 cells, at least 1 million, at least 5 million, at least 10 million, at least 50 million, at least 100 million, at least 200 million, at least 300 million, at least 400 million, at least 500 million, at least 600 million, at least 700 million, at least 800 million, at least 900 million, or at least 1000 million cells. The number of cells introduced into the lumen of the device may vary and may be determined empirically.

In some embodiments, the cells introduced into the lumen of the device may be modified. Modifications can include genetic modifications or epigenetic modifications. Genetic modifications can include insertions, deletions, mutations, or inversions. Modifications can be introduced into the cells through gene editing, for example, through the use of a CRISPR/Cas gene editing system. The cells can be modified to alter (e.g., increase, decrease, or eliminate) expression of a protein of interest. The cells can be modified to increase expression of a protein of interest. Genetic modification to alter expression of the protein of interest can comprise modifications to regulatory regions involved in the expression of the protein of interest. Regulatory regions can include, but are not limited to promoters, transcription factors, enhancers, silencers, 5'UTR, and 3'UTR. The cells can be modified to produce a structural or functional variant of a protein of interest. The cells introduced into the lumen can be recombinant cells.

In some aspects, the cells may be therapeutic cells, for example, cells which produce and release or secrete therapeutic molecules that provide a therapeutic benefit to the biological subject. Generally, the device may be configured such that therapeutic molecules may be released from the device to e.g., effectuate a therapeutic response in the biological subject. The therapeutic molecule can be a protein. In some cases, the protein can be a naturally-produced protein. In some cases, the protein can be a recombinant protein. The protein can be a hormone or an enzyme. The protein can be insulin or an analog thereof, erythropoietin, a coagulation modulator (e.g. coagulant or anticoagulant), a thrombolytic enzyme, an alpha-galactosidase A, deoxyribonuclease I, glucocerebrosidase, iduronidase, N-acetylgalactosamine-4-sulfatase, growth hormone or an analog thereof, a gonadotropin, calcitonin, glucagon, an interferon, an interleukin, thrombopoietin, estrogen, testosterone, or a granulocyte colony-stimulating factor. The coagulant can be Factor-VII A, Factor-VIII, or Factor-IX. The gonadotrophin can be a follitropin, a choriogonadotropin, follicle-stimulating hormone, or luteinizing hormone. The interferon can be an interferon-alpha, an interferon-beta, or a interferon-gamma. The interleukin can be IL-1, IL-2, IL-3, IL-4, IL-10, IL-11, IL-13, IL-15, IL-17, or IL-18. In some cases, the therapeutic molecule is an antibody. In one non-limiting example, the cells are islets or islet cells, and the therapeutic molecule is insulin (e.g., to treat a diabetic subject). The type of cell to be used in the device generally is determined based on the desired therapeutic molecule and the therapeutic response desired in the subject.

In some aspects, the devices provided herein may be configured to be implanted into a subject. In some cases, the device may be used to transplant or transfer cells into a subject. In some cases, the devices may be implanted into a subject by subcutaneous implantation, intramuscular implantation, subdermal implantation, intraperitoneal implantation, retroperitoneal implantation, omentum implantation, implantation into the brain, subfacial implantation, implantation into the bone, ocular implantation, implantation into an organ, implantation adjacent to an organ (e.g., liver, etc.). The site of implantation may be selected based on the diseased/injured tissue that requires treatment. For treatment of a disease such as diabetes mellitus (DM), the device may be placed in a clinically convenient site such as the subcutaneous space or the omentum.

In certain embodiments, the device may be sealed entirely along the edges of the device thereby forming a completely enclosed internal space or lumen. In other embodiments, the device may be open at one or more locations at an edge of the device allowing access to the internal space or lumen of the device. When the device includes two openings into the lumen of the device, the two openings may be located opposite each other, such as, on opposite sides of the planar device. In some cases, a device of the disclosure does not include a support or frame. For example, the first polymer layer and the second polymer layer may be laid on top of one another and sealed along the periphery of the layers. In some cases, a device of the disclosure may have a support or frame. In such embodiments, the device may be flexible or have a flexible structure such that it can be rolled up. In some cases, the device may be folded or rolled up prior to implantation, and then unfolded or unrolled after implantation.

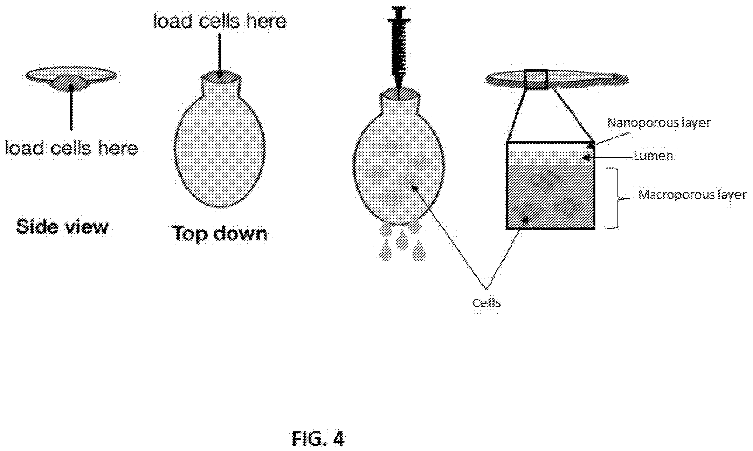

In some aspects, the cells may be loaded into the lumen of the device prior to implantation into a subject. In some cases, after the cells have been loaded into the lumen of the device, the cells may embed within the macroporous scaffold of the second polymer layer **(****FIG. 4****).** In other aspects, the device (without any cells loaded therein) may be implanted into the subject. The device may then later be loaded with cells, after the device has been implanted for a period of time. For example, the device may be implanted into a subject at least 1 day, at least 2 days, at least 3 days, at least 4 days, at least 5 days, at least 6 days, at least 1 week, at least 1.5 weeks, at least 2 weeks, at least 2.5 weeks, at least 3 weeks, at least 3.5 weeks, or at least 4 weeks prior to loading the device with cells. In some aspects, the device may further comprise a port for loading cells into the device. In some cases, the port is a tubing for introducing cells into the lumen of the device. In certain embodiments, the tubing may be affixed to the device by sealing the first and second layers, at the opening, to the exterior wall of the tubing. In certain embodiments, a first end of the tubing may be placed at the opening and positioned in the lumen such that a minimal volume of the lumen is taken up by the tubing while allowing loading of fluids into the lumen of the device. The second end of the tubing, which is distal to the first end, may be used as a port for introducing fluids into the lumen via the tubing. The length of the tubing may be selected based on a number of factors. For example, a shorter tubing may be used when loading a population of cells into the lumen of the device *ex vivo,* for example, prior to placement of the device into a subject. A longer tubing may be used if the tubing is to be used for introducing fluids into and/or removing fluids from the lumen of the device after placement of the device into a subject. In some cases, the port may be accessible after the device has been implanted into the subject, such that the device may be pre-implanted without any cells contained within the lumen, and the lumen may be loaded with cells at some period of time after implantation. In some aspects, the device may be pre-loaded with support cells or factors (e.g., growth factors) prior to introduction of the therapeutic cells.

The devices herein may be designed such that therapeutic molecules (e.g., such as those produced by and secreted from therapeutic cells) can diffuse out of the lumen or internal compartment of the device. Additionally, the devices may allow molecules present outside of the device to permeate into the lumen or internal compartment of the device. In some cases, the device may allow for oxygen to diffuse into and/or out of the device. In some aspects, the devices may promote the growth of blood vessels into or around the implantation site of the device (e.g., promote vascularization of the device).

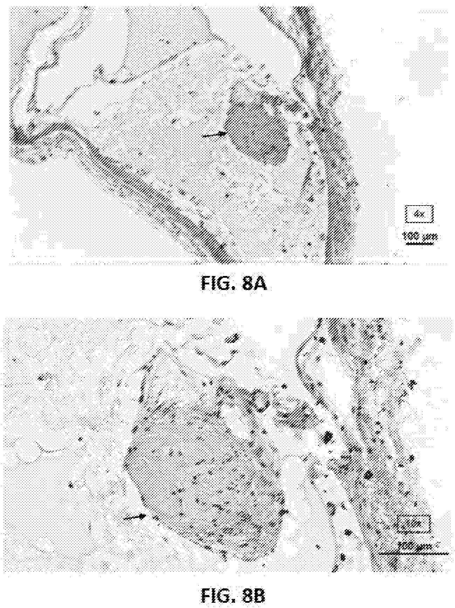

When an implantable device containing cells has been isolated from the immune system by encasing it in a cell-impermeable layer, the implantable device often stimulates a local inflammatory response, called the foreign body response (FBR) that has long been recognized as limiting the function of implanted devices that require solute transport. FBR has been well described in the literature and is composed of three main layers. The innermost FBR layer, adjacent to an implanted device is composed of macrophages and foreign body giant cells. These cells form a monolayer of closely opposed cells over the surface of an implanted device. The intermediate FBR layer, lying distal to the first layer with respect to the device, is a wide zone (30-100 microns) composed primarily of fibroblasts and fibrous matrix. The outermost FBR layer is loose connective granular tissue containing new blood vessels. Upon induction of a FBR, an implanted device is isolated from the *in vivo* environment limiting the exchange of molecules with the implanted device, limiting the utility of the implanted device as well as, leading to the death of any cells provided within the implanted device. In some cases, a device of the disclosure may not provoke a foreign body response, may not provoke a substantial foreign body response, or may provoke a limited foreign body response, such that the cells encapsulated within the device remain viable for a period of time. In some cases, a foreign body response may be evidenced by fibrosis around the implanted device. In some cases, a device of the disclosure may not provoke fibrosis, may not provoke substantial fibrosis, or may provoke limited fibrosis, around the implanted device. **FIGS. 8A** and **8B** depict a device of the disclosure demonstrating a lack of fibrosis around the implanted device.

In some aspects, the devices disclosed herein may support viability of cells present in the lumen of the device upon transplantation into a subject, for at least one week, two weeks, three weeks, four weeks, one month, at least 2 months, at least 3 months, at least 4 months, at least 5 months, at least 6 months, at least 7 months, at least 8 months, at least 9 months, at least 10 months, at least 11 months, at least 12 months, at least 2 years, at least 3 years, at least 4 years, at least 5 years, or longer.

In some aspects, the devices disclosed herein may be substantially impermeable to cells present outside the device such that cells present outside the device do not enter the device. For example, in some cases, the device is substantially impermeable to immune cells such that immune cells present outside the device may not enter the lumen of the device. In some aspects, the devices disclosed herein may be substantially impermeable to molecules present outside the device such that said molecules do not enter the device. For example, in some cases, the device is substantially impermeable to immunoglobulins such that the concentration of any immunoglobulins that gain access to the lumen of the device is below the level needed for an immune response against the cells present inside the lumen of the device. In another example, the devices of the present disclosure may substantially limit cytokines from entering the lumen of the device such that the concentration of cytokines that gain access to the lumen of the device is below the level required for an immune response against the cells present inside the lumen of the device. In some aspects, the devices are configured to promote nutrient exchange with the encapsulated cells and/or diffusion of metabolic waste products out of the device. In some cases, the devices provided herein are configured to permit the passage of small molecules (including therapeutic molecules), but to substantially inhibit passage of large molecules.

In some aspects, the exterior surface of the device may be modified by coating with one or more agents that improve a function of the device. For example, molecules that promote vascularization of the device or inhibit immune or inflammatory responses to the device may be disposed on the exterior of the device. In some cases, agents may be disposed within the device (e.g., loaded inside the lumen of the device, or coated on an interior surface of the device. In some cases, the agents may diffuse out of the lumen of the device and act on an external environment. Additionally or alternatively, the agents inside the lumen of the device may act on cells disposed within the lumen of the device (e.g., maturation factors that act on partially mature stem cells). Such molecules or agents may include, but are not limited, to VEGF (vascular endothelial growth factor), PDGF (platelet-derived growth factor), FGF-1 (fibroblast growth factor), angiopoietin MCP-1, αᵥβ₃, αᵥβ₅, CD-31, VE-cadherin, ephrin, plasminogen activators, angiogenin, Del-1, aFGF (acid fibroblast growth factor), vFGF (basic fibroblast growth factor), follistatin, G-CSF (granulocyte colony-stimulating factor), HGF (hepatocyte growth factor), Leptin, placental growth factor, PD-ECGF (platelet-derived endothelial growth factor), and the like.

Further provided herein are methods for using the subject devices. In one aspect, a device of the disclosure may be implanted into a subject. In some cases, the device may be implanted into the subject with cells pre-loaded into the lumen of the device. In other cases, the device may be implanted into the subject without cells pre-loaded into the lumen of the device. In such cases, the device may, after a period of time, be loaded with cells (e.g., by using a pre-implantation port or tubing of the device). In some cases, the methods may involve using the subject devices for transplanting cells that produce and secrete therapeutic molecules into a subject. In some cases, the therapeutic molecules may diffuse out of the device and into an environment of the subject, such that the therapeutic molecule has a therapeutic effect on the subject.

In some cases, the methods may be used to treat a disease or disorder in the subject. The disease or disorder may be a disease or disorder characterized by lack of functional cells or by deficient production of a protein. In some case, the disease or disorder can be, without limitation, diabetes, anemia, a clotting disorder (e.g., hemophilia), cardiovascular disease, Fabry disease, Gaucher disease, mucopolysaccharidosis, growth hormone deficiency, infertility, osteoporosis, hypoglycemia, Parkinson's disease, or muscular dystrophy .

In a non-limiting example, the methods may be used to transplant islets or islet cells into a subject, wherein the islets or islet cells produce and secrete insulin. In such cases, the methods may be used to treat a subject having or suspected of having diabetes. The diabetes can be type 1 diabetes. The device may include pancreatic islet cells or may include stem cells that are capable of differentiating into pancreatic islet cells. In certain embodiments, pluripotent stem cells (PSCs) may be differentiated into pancreatic islet cells inside the device and then the device containing the differentiated pancreatic islet cells is placed in the subject (e.g., in the omentum, adjacent to pancreas or liver). In some case, the device may include PSCs and the device may be implanted adjacent the pancreas or liver of the subject.

### EXAMPLES

### Example 1. Cell encapsulation device.

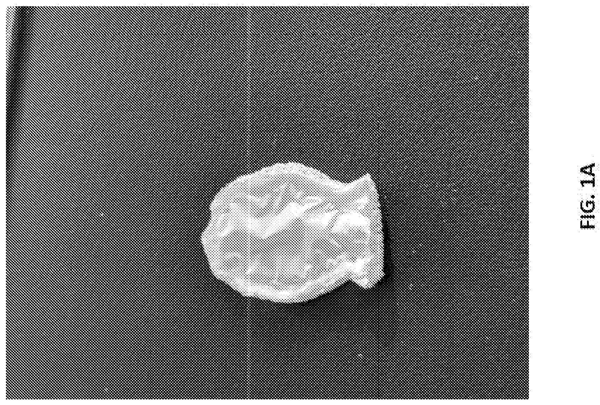

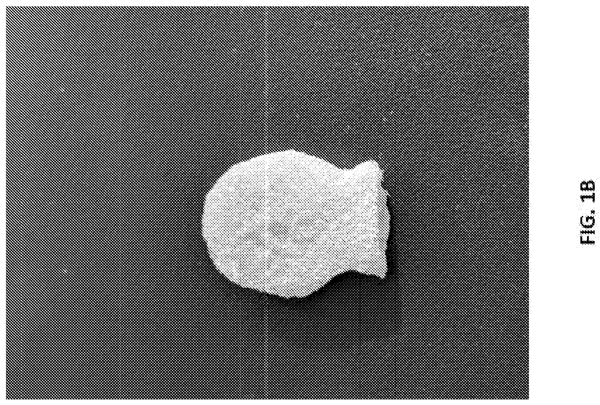

**FIG. 1A** and **FIG. 1B** depict a non-limiting example of a cell encapsulation device according to embodiments of the disclosure. **FIG. 1A** depicts a first polymer layer. The first polymer layer comprises a plurality of pores having an average connectivity diameter of about 200 nm, and an average pore size of about 2 µm. FIG. 1B depicts a second polymer layer. The second polymer layer comprises a plurality of pores having an average pore diameter of about 500 µm to about 3 mm, and an average connectivity diameter of about 100 µm to about 500 µm.

### Example 2. Polymer scaffold

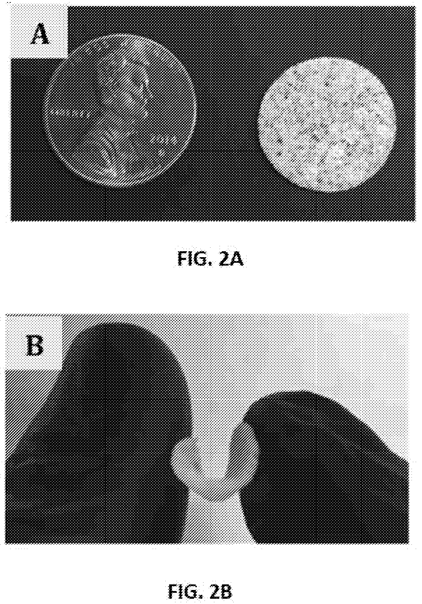

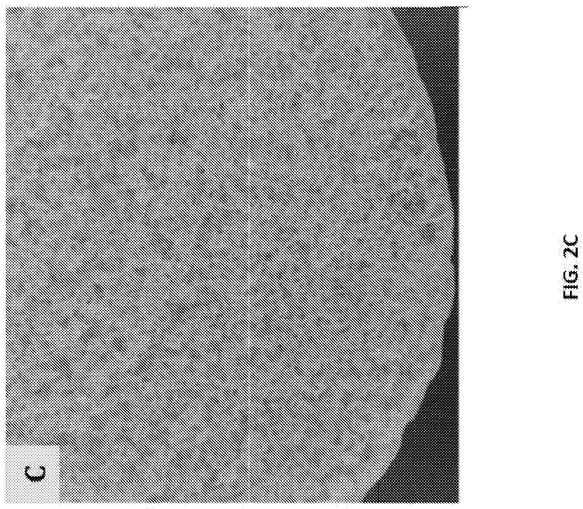

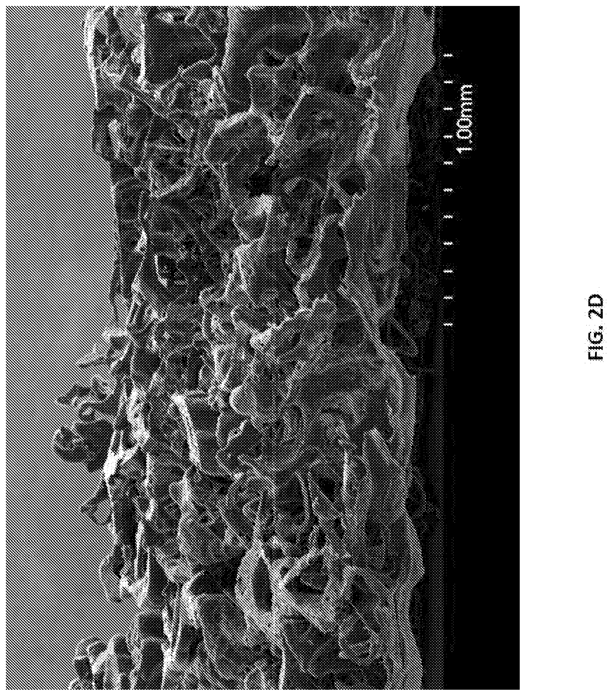

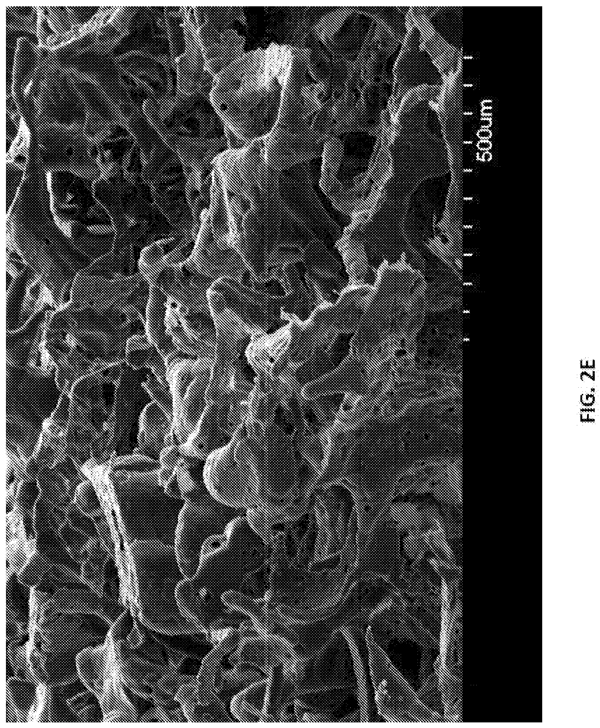

A three-dimensional polymer scaffold was designed to facilitate the transplantation of islets in a manner that permits optimal packing of islets with better spatial distribution. The scaffolds were designed with stochastic macroporous structures that served the function of allowing islets to embed within as well as the ingrowth of blood vessels to vascularize the islets **(****FIGS. 2A-2E****).**

### Example 3. Cell encapsulation device.

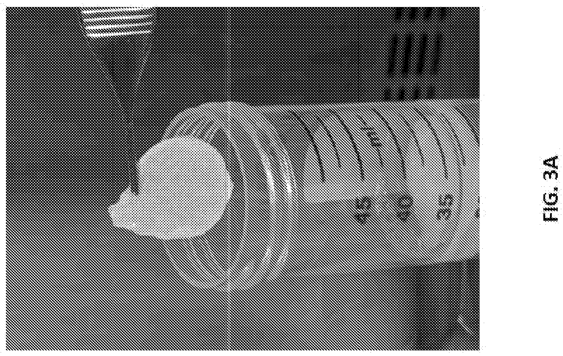

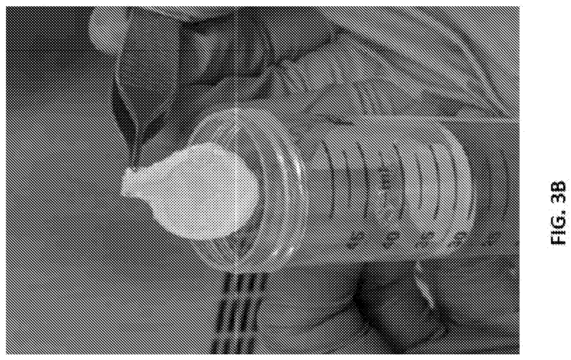

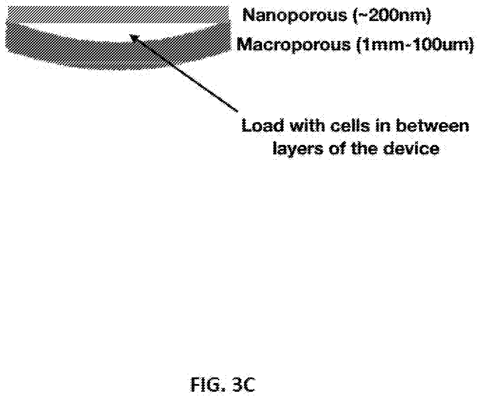

Devices were constructed with a nanoporous layer and a macroporous layer (e.g., a scaffold according to Example 2) constructed of polycaprolactone **(****FIGS. 3A-3C****).** The nanoporous membrane was ~20 µm thick and consisted of pores having a pore diameter of ~2 µm with a connectivity diameter of ~200 nm. The macroporous membrane was ~500 µm thick and consisted of macroscopic pores with a connectivity diameter and a pore diameter of 200-400 µm. To create the device, the macroporous and nanoporous membranes were overlaid and directly sealed along the perimeter.

### Example 4. Syngeneic islet implantation into diabetic mice

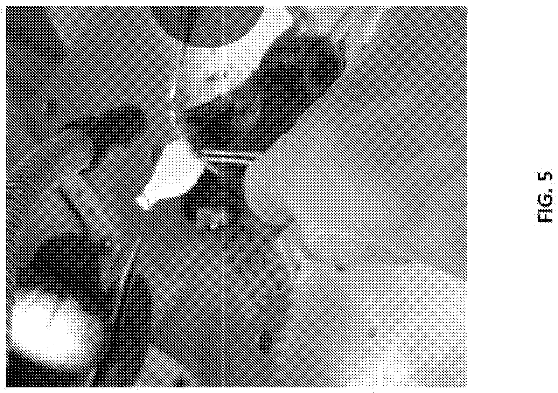

Devices, as described in Example 3, were loaded with about 500 syngeneic C57BL/6 islets, sealed, and implanted subcutaneously in C57BL/6 mice **(****FIG. 5****).** At 21 days, an intraperitoneal glucose tolerance test (IPGTT) was conducted. The glucose tolerance test measured the clearance of an intraperitoneally injected glucose load from the body, and can be used to detect disturbances in glucose metabolism that can be linked to human conditions such as diabetes or metabolic syndrome. Animals were fasted for approximately 16 hours, and fasted blood glucose levels were determined before a solution of glucose was administered by intra-peritoneal (IP) injection. Subsequently, the blood glucose level was measured at different time points during the following 2 hours.

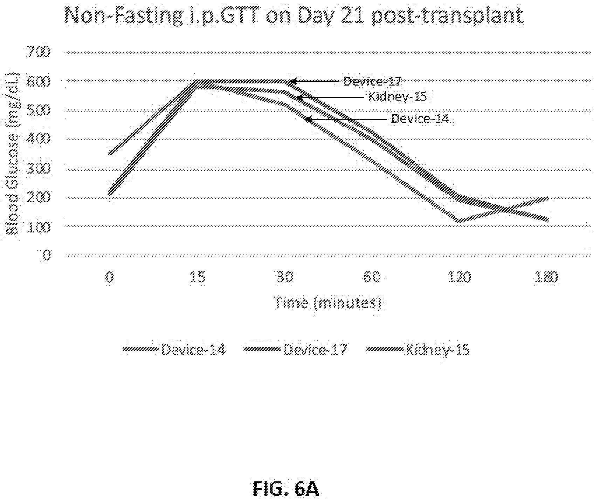

IPGTT was conducted at 21 days, with 500 islets encapsulated within the device implanted subcutaneously as well as 500 islets implanted directly into the kidney capsule. Islets in the device followed the same glucose curve as islets within the kidney capsule, which indicated a similar glucose response **(****FIG. 6A****).**

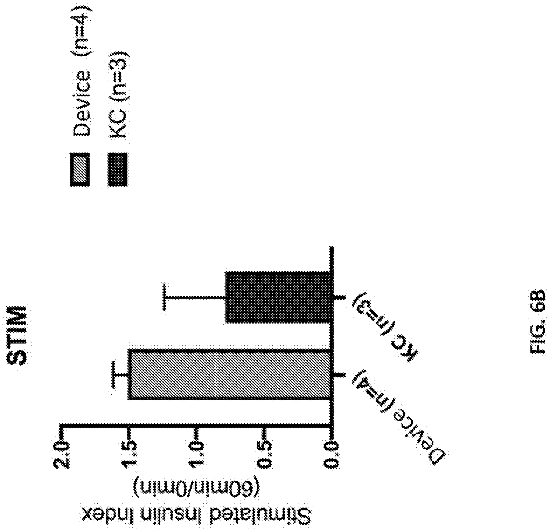

Additionally, insulin secretion was analyzed using a glucose-stimulated insulin secretion assay. The glucose stimulation index is a metric to quantify beta cell function by comparing the ratio of insulin release in a high glucose state relative to a resting state. The ratio of insulin secreted at high to low glucose conditions was used to calculate the glucose stimulation index. As depicted in **FIG. 6B****,** islets that were encapsulated within a device as described in Example 3 had a greater glucose stimulation index than islets that were implanted directly into the kidney capsule (KC).

### Example 5. Assessment of device post-engraftment

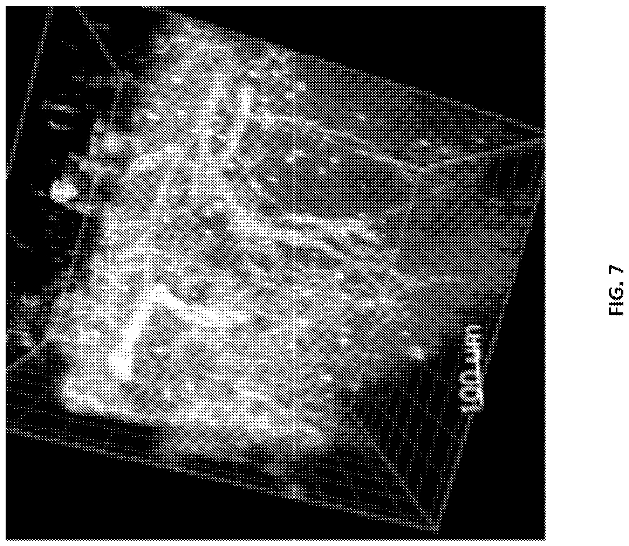

Devices were implanted subcutaneously for greater than 30 days to allow engraftment. Post-sacrifice, tissue was excised and fixed using Z-Fix for greater than 24 hours at 4°C, then washed in PBS. The tissue and the device were imaged using CLARITY (Clear Lipid-exchanged Acrylamide-hybridized Rigid Imaging / Immunostaining / in situ-hybridization-compatible Tissue hydrogel), a technique which embeds tissue in a hydrogel matrix. Lipids are cleared to create an optically transparent hybrid that is transparent to light and permeable to molecules. Markers included were DAPI for nuclear staining, and CD31 and alpha-smooth muscle action (α-SMA) to stain blood vessels. **FIG. 7** depicts staining of blood vessels that have penetrated into and around the device.

**FIG. 8A** and **FIG. 8B** depict immunohistochemical staining of sections of the device after engraftment in a mouse. The figures depict islets embedded within the macroporous scaffold layer of the device (arrows).

While preferred embodiments of the present invention have been shown and described herein, it will be obvious to those skilled in the art that such embodiments are provided by way of example only. Numerous variations, changes, and substitutions will now occur to those skilled in the art without departing from the scope of the appended claims.

## Claims

1. A cell encapsulation device comprising:
a) a first polymer layer comprising a plurality of pores and one or more channels, wherein said one or more channels connect two adjacent pores of said plurality of pores, and wherein said one or more channels comprise an average connectivity diameter of less than about 500 nm;
b) a second polymer layer comprising a plurality of pores and one or more channels, wherein said one or more channels connect two adjacent pores of said plurality of pores, and wherein said one or more channels comprise an average connectivity diameter of greater than about 100 µm;
wherein said first polymer layer and said second polymer layer define a lumen for enclosing a plurality of cells.

2. The cell encapsulation device of claim 1, wherein said one or more channels in said first polymer layer have an average connectivity diameter from about 10 nm to about 200 nm.

3. The cell encapsulation device of claim 1 or 2, wherein said one or more channels in said second polymer layer have an average connectivity diameter from about 100 µm to about 500 µm, preferably from about 200 µm to about 400 µm.

4. The cell encapsulation device of any one of claims 1-3, wherein said plurality of pores present in said first polymer layer have an average pore diameter from about 1 µm to about 5 µm.

5. The cell encapsulation device of any one of claims 1-4, wherein said plurality of pores present in said second polymer layer have an average pore diameter from about 100 µm to about 3 mm, preferably from about 100 µm to about 1 mm.

6. The cell encapsulation device of any one of claims 1-5, wherein said first polymer layer has a thickness from about 10 µm to about 200 µm, preferably from about 20 µm to about 50 µm, more preferably from about 18 µm to about 22 µm.

7. The cell encapsulation device of any one of claims 1-6, wherein said second polymer layer has a thickness from about 1 mm to about 5 mm.

8. The cell encapsulation device of any one of claims 1-7, further comprising a third polymer layer comprising one or more channels having an average connectivity diameter of less than about 500 nm.

9. The cell encapsulation device of any one of claims 1-8, further comprising a port for introducing said plurality of cells into said lumen of said device.

10. The cell encapsulation device of any one of claims 1-9, wherein said first polymer layer, said second polymer, said third polymer layer, or any combination thereof, comprises a biocompatible polymer selected from the group consisting of: polycaprolactone (PCL), polyvinylidene fluoride (PVDF), polytetrafluoroethylene (PTFE), polyethylene (PE), methacrylate polymer, polyethyleneimine, polyethyleneimine-dextran sulfate, poly(vinylsiloxane) ecopolymerepolyethyleneimine, phosphorylcholine, poly(ethyl methacrylate), polyurethane, poly(ethylene glycol) (PEG), poly(lactic-glycolic acid) (PLGA), hydroxyapatite, poly(lactic acid), polyhydroxyvalerte and copolymers thereof, polyhydroxybutyrate and copolymers thereof, polydiaxanone, polyanhydride, polycyanocrylate, poly(amino acids), poly(orthoesters), polyesters, collagen, gelatin, cellulose polymer, chitosans, alginates, laminin, and any combination thereof.

11. The cell encapsulation device of claim 10, wherein said biocompatible polymer is polycaprolactone.

12. The cell encapsulation device of any one of claims 1-11, wherein said plurality of cells are selected from the group consisting of: thyroid cells, parathyroid cells, bone marrow cells, mesenchymal stem cells, stromal cells, pluripotent stem cells, induced pluripotent stem cells, embryonic stem cells, blood vessel cells, cells derived from adipose tissue, cells derived from bone marrow, intestinal cells or cells derived therefrom, islets or islet cells, Sertoli cells, beta cells, progenitors of islet cells, progenitors of beta cells, peripheral blood progenitor cells, stem cells or derivatives thereof isolated from adult tissue, retinal progenitor derivative cells, cardiac progenitor derivative cells, osteoprogenitor cells, neuronal progenitor cells, genetically transformed cells, and any combination thereof.

13. The cell encapsulation device of any one of claims 1-12, wherein said first polymer layer and said second polymer layer are directly sealed along a periphery of said first polymer layer and said second polymer layer.

14. The cell encapsulation device of any one of claims 1-13, wherein said device does not comprise a support or frame.

15. The cell encapsulation device of any one of claims 1-14, wherein said first polymer layer, said second polymer layer, or both, are non-laminated polymer layers.

## Patentansprüche

1. Zelleinkapselungsvorrichtung, umfassend:
a) eine erste Polymerschicht, umfassend eine Vielzahl von Poren und einen oder mehrere Kanäle, wobei der eine oder die mehreren Kanäle zwei benachbarte Poren der Vielzahl von Poren verbinden, und wobei der eine oder die mehreren Kanäle einen mittleren Konnektivitäts-Durchmesser von weniger als etwa 500 nm umfassen;
b) eine zweite Polymerschicht, umfassend eine Vielzahl von Poren und einen oder mehrere Kanäle, wobei der eine oder die mehreren Kanäle zwei benachbarte Poren der Vielzahl von Poren verbinden, und wobei der eine oder die mehreren Kanäle einen mittleren Konnektivitäts-Durchmesser von mehr als etwa 100 µm umfassen;
wobei die erste Polymerschicht und die zweite Polymerschicht ein Lumen zum Einschließen einer Vielzahl von Zellen definieren.

2. Zelleinkapselungsvorrichtung nach Anspruch 1, wobei der eine oder die mehreren Kanäle in der ersten Polymerschicht einen mittleren Konnektivitäts-Durchmesser von etwa 10 µm bis etwa 200 µm aufweisen.

3. Zelleinkapselungsvorrichtung nach Anspruch 1 oder 2, wobei der eine oder die mehreren Kanäle in der zweiten Polymerschicht einen mittleren Konnektivitäts-Durchmesser von etwa 100 µm bis etwa 500 µm aufweisen, vorzugsweise von etwa 200 µm bis etwa 400 µm.

4. Zelleinkapselungsvorrichtung nach Anspruch 1 bis 3, wobei die Vielzahl von Poren in der ersten Polymerschicht einen mittleren Poren-Durchmesser von etwa 1 µm bis etwa 5 µm aufweist.

5. Zelleinkapselungsvorrichtung nach einem der Ansprüche 1 bis 4, wobei die Vielzahl von Poren in der zweiten Polymerschicht einen mittleren Poren-Durchmesser von etwa 100 µm bis etwa 3 mm aufweist, vorzugsweise von etwa 100 µm bis etwa 1 mm.

6. Zelleinkapselungsvorrichtung nach einem der Ansprüche 1 bis 5, wobei die erste Polymerschicht eine Dicke von etwa 10 µm bis etwa 200 µm aufweist, vorzugsweise von etwa 20 µm bis etwa 50 µm, noch mehr vorzuziehen von etwa 18 µm bis etwa 22 µm.

7. Zelleinkapselungsvorrichtung nach einem der Ansprüche 1 bis 6, wobei die zweite Polymerschicht eine Dicke von etwa 1 mm bis etwa 5 mm aufweist.

8. Zelleinkapselungsvorrichtung nach einem der Ansprüche 1 bis 7, ferner umfassend eine dritte Polymerschicht, die einen oder mehrere Kanäle mit einem mittleren Konnektivitäts-Durchmesser von weniger als etwa 500 nm umfasst.

9. Zelleinkapselungsvorrichtung nach einem der Ansprüche 1 bis 8, ferner umfassend einen Anschluss zum Einführen der Vielzahl von Zellen in das Lumen der Vorrichtung.

10. Zelleinkapselungsvorrichtung nach einem der Ansprüche 1 bis 9, wobei die erste Polymerschicht, die zweite Polymerschicht, die dritte Polymerschicht oder jede Kombination davon ein biokompatibles Polymer umfasst, ausgewählt aus der Gruppe bestehend aus: Polycaprolacton (PCL), Polyvinylidenfluorid (PVDF), Polytetrafluorethylen (PTFE), Polyethylen (PE), Methacrylatpolymeren, Polyethylenimin, Polyethylenimin-Dextransulfat, Poly(vinylsiloxan), Copolymeren von Polyethylenimin, Phosphorylcholin, Poly(ethylmethacrylat), Polyurethan, Poly(ethylenglykol) (PEG), Poly(milchsäure-co-glykolsäure) (PLGA), Hydroxylapatit, Polymilchsäure, Polyhydroxyvalerat und Copolymere davon, Polyhydroxybutyrat und Copolymere davon, Polydioxanon, Polyanhydriden, Polycyanoacrylaten, Poly(aminosäuren), Poly(orthoestern), Polyestern, Kollagen, Gelatine, Cellulosepolymeren, Chitosanen, Alginaten, Laminin und jeder Kombination davon.

11. Zelleinkapselungsvorrichtung nach Anspruch 10, wobei das biokompatible Polymer Polycaprolacton ist.

12. Zelleinkapselungsvorrichtung nach einem der Ansprüche 1 bis 11, wobei die Vielzahl von Zellen ausgewählt ist aus der Gruppe bestehend aus: Schilddrüsenzellen, Nebenschilddrüsenzellen, Knochenmarkzellen, mesenchymalen Stammzellen, Stromazellen, pluripotenten Stammzellen, induzierten pluripotenten Stammzellen, embryonalen Stammzellen, Blutgefäßzellen, aus Fettgewebe abgeleiteten Zellen, aus Knochenmark abgeleiteten Zellen, Darmzellen oder davon abgeleiteten Zellen, Langerhans-Inseln oder Inselzellen, Sertoli-Zellen, Betazellen, Vorläuferzellen von Inselzellen, Vorläuferzellen von Betazellen, peripheren Blutvorläuferzellen, aus adultem Gewebe isolierten Stammzellen oder Derivaten davon, von retinalen Vorläuferzellen abgeleiteten Zellen, von kardialen Vorläuferzellen abgeleiteten Zellen, Osteoprogenitorzellen, neuronalen Vorläuferzellen, genetisch transformierten Zellen und jeder Kombination davon.

13. Zelleinkapselungsvorrichtung nach einem der Ansprüche 1 bis 12, wobei die erste Polymerschicht und die zweite Polymerschicht direkt entlang einer Peripherie der ersten Polymerschicht und der zweiten Polymerschicht versiegelt sind.

14. Zelleinkapselungsvorrichtung nach einem der Ansprüche 1 bis 13, wobei die Vorrichtung keine Stützstruktur und keinen Rahmen umfasst.

15. Zelleinkapselungsvorrichtung nach einem der Ansprüche 1 bis 14, wobei die erste Polymerschicht, die zweite Polymerschicht oder beide nicht-laminierte Polymerschichten sind.

## Revendications

1. Dispositif d'encapsulation de cellule comprenant :
a) une première couche de polymère comprenant une pluralité de pores et un ou plusieurs canaux, dans lequel lesdits un ou plusieurs canaux relient deux pores adjacents de ladite pluralité de pores, et dans lequel lesdits un ou plusieurs canaux comprennent un diamètre de connectivité moyen inférieur à environ 500 nm ;
b) une deuxième couche de polymère comprenant une pluralité de pores et un ou plusieurs canaux, dans lequel lesdits un ou plusieurs canaux relient deux pores adjacents de ladite pluralité de pores, et dans lequel lesdits un ou plusieurs canaux comprennent un diamètre de connectivité moyen supérieur à environ 100 µm ;
dans lequel ladite première couche de polymère et ladite deuxième couche de polymère définissent une lumière destinée à enfermer une pluralité de cellules.

2. Dispositif d'encapsulation de cellule selon la revendication 1, dans lequel lesdits un ou plusieurs canaux dans ladite première couche de polymère présentent un diamètre de connectivité moyen d'environ 10 nm à environ 200 nm.

3. Dispositif d'encapsulation de cellules selon la revendication 1 ou 2, dans lequel lesdits un ou plusieurs canaux dans ladite deuxième couche de polymère présentent un diamètre de connectivité moyen d'environ 100 µm à environ 500 µm, de préférence d'environ 200 µm à environ 400 µm.

4. Dispositif d'encapsulation de cellules selon l'une quelconque des revendications 1 à 3, dans lequel ladite pluralité de pores présents dans ladite première couche de polymère présente un diamètre moyen de pores d'environ 1 µm à environ 5 µm.

5. Dispositif d'encapsulation de cellules selon l'une quelconque des revendications 1 à 4, dans lequel ladite pluralité de pores présents dans ladite deuxième couche de polymère présente un diamètre moyen de pores d'environ 100 µm à environ 3 mm, de préférence d'environ 100 µm à environ 1 mm.

6. Dispositif d'encapsulation de cellule selon l'une quelconque des revendications 1 à 5, dans lequel ladite première couche de polymère a une épaisseur d'environ 10 µm à environ 200 µm, de préférence d'environ 20 µm à environ 50 µm, plus préférentiellement d'environ 18 µm à environ 22 µm.

7. Dispositif d'encapsulation de cellule selon l'une quelconque des revendications 1 à 6, dans lequel ladite deuxième couche de polymère présente une épaisseur d'environ 1 mm à environ 5 mm.

8. Dispositif d'encapsulation de cellule selon l'une quelconque des revendications 1 à 7, comprenant en outre une troisième couche de polymère comprenant un ou plusieurs canaux présentant un diamètre de connectivité moyen inférieur à environ 500 nm.

9. Dispositif d'encapsulation de cellules selon l'une quelconque des revendications 1 à 8, comprenant en outre un orifice destiné à introduire ladite pluralité de cellules dans ladite lumière dudit Dispositif.

10. Dispositif d'encapsulation de cellule selon l'une quelconque des revendications 1 à 9, dans lequel ladite première couche de polymère, ladite deuxième couche de polymère, ladite troisième couche de polymère, ou toute combinaison de ceux-ci, comprend un polymère biocompatible sélectionné dans le groupe constitué des éléments suivants : polycaprolactone (PCL), fluorure de polyvinylidène (PVDF), polytétrafluoroéthylène (PTFE), polyéthylène (PE), polymère de méthacrylate, polyéthylèneimine, polyéthylèneimine-dextrane sulfate, poly(vinylsiloxane) ecopolymerepolyéthylèneimine, phosphorylcholine, poly(méthacrylate d'éthyle), polyuréthane, poly(éthylène glycol) (PEG), poly(acide lactique-glycolique) (PLGA), hydroxyapatite, poly(acide lactique), polyhydroxyvalerte et ses copolymères, polyhydroxybutyrate et ses copolymères, polydiaxanone, polyanhydride, polycyanocrylate, poly(acides aminés), poly(orthoesters), polyesters, collagène, gélatine, polymère de cellulose, chitosanes, alginates, laminine, et toute combinaison de ceux-ci.

11. Dispositif d'encapsulation de cellules selon la revendication 10, dans lequel ledit polymère biocompatible est la polycaprolactone.

12. Dispositif d'encapsulation de cellules selon l'une quelconque des revendications 1 à 11, dans lequel ladite pluralité de cellules est sélectionnée dans le groupe constitué des éléments suivants : cellules thyroïdiennes, cellules parathyroïdiennes, cellules de moelle osseuse, cellules souches mésenchymateuses, cellules stromales, cellules souches pluripotentes, cellules souches pluripotentes induites, cellules souches embryonnaires, cellules de vaisseaux sanguins, cellules dérivées du tissu adipeux, cellules dérivées de la moelle osseuse, cellules intestinales ou cellules dérivées de celles-ci, cellules d'îlots ou d'îlots, cellules de Sertoli, cellules bêta, progéniteurs de cellules d'îlot, progéniteurs de cellules bêta, cellules progénitrices du sang périphérique, cellules souches ou leurs dérivés isolés du tissu adulte, cellules dérivées de progéniteurs rétiniens, cellules dérivées de progéniteurs cardiaques, cellules ostéoprogénitrices, cellules progénitrices neuronales, cellules génétiquement modifiées, et toute combinaison de celles-ci.

13. Dispositif d'encapsulation de cellule selon l'une quelconque des revendications 1 à 12, dans lequel ladite première couche de polymère et ladite deuxième couche de polymère sont scellées directement le long d'une périphérie de ladite première couche de polymère et de ladite deuxième couche de polymère.

14. Dispositif d'encapsulation de cellules selon l'une quelconque des revendications 1 à 13, dans lequel ledit Dispositif ne comprend pas de support ou de cadre.

15. Dispositif d'encapsulation de cellule selon l'une quelconque des revendications 1 à 14, dans lequel ladite première couche de polymère, ladite deuxième couche de polymère, ou les deux, présentent des couches de polymère non stratifiées.
